# EUROPEAN PATENT APPLICATION

(11) **EP 4 603 524 A1**
(43) Date of publication of application: **20.08.2025**
(21) Application number: 23877385.7
(22) Date of filing: 16.10.2023
(51) Int. Cl.: C08G 18/38, G02B 1/04

(54) **POLYTHIOL COMPOSITION, POLYMERIZABLE COMPOSITION, RESIN, MOLDED BODY, OPTICAL MATERIAL, AND LENS**

(30) Priority: 14.10.2022 JP 2022165813
(71) Applicant: Mitsui Chemicals, Inc., Tokyo 104-0028 (JP)
(72) Inventor: NAKANO, Shotaro, Omuta-shi, Fukuoka 836-8610 (JP); MURAKAMI, Masakazu, Omuta-shi, Fukuoka 836-8610 (JP)
(74) Representative: Mewburn Ellis LLP
(86) International application number: PCT/JP2023/037453
(87) International publication number: WO 2024/080384

(57) **Abstract**

A polythiol composition containing a polythiol compound (A) that is 4-mercaptomethyl-1,8-dimercapto-3,6-dithiaoctane and a compound represented by Formula (1), wherein, in a high-performance liquid chromatography measurement, a peak area of the compound represented by the Formula (1) is 11.00 or less with respect to a total peak area 100 of compounds contained in the polythiol composition.

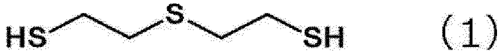

## Description

### TECHNICAL FIELD

The present disclosure relates to a polythiol composition, a polymerizable composition, a resin, a molded body, an optical material, and a lens.

### BACKGROUND ART

Plastic lenses are lighter and less likely to break than inorganic lenses, and can be dyed. Thus, in recent years, plastic lenses have rapidly become widespread in spectacle lenses and optical elements such as camera lenses.

Resins for plastic lenses have been required to have higher performance, and have been required to have a higher refractive index, a higher Abbe number, a lower specific gravity, higher heat resistance, and the like. Various resin materials for lenses have been developed and used so far.

For example, Patent Document 1 describes a mercapto compound represented by a specific structural formula.

For example, Patent Document 2 describes a method of producing a polythiol compound including: a process of reacting 2-mercaptoethanol with an epihalohydrin compound represented by a specific Formula (1) at a temperature of from 10 to 50°C to obtain a polyalcohol compound represented by a specific Formula (2); a process of reacting the obtained polyalcohol compound represented by Formula (2) with thiourea in the presence of hydrogen chloride to obtain an isothiuronium salt; a process of adding ammonia water to a reaction liquid containing the obtained isothiuronium salt within 80 minutes while maintaining the reaction liquid at a temperature of from 15 to 60°C to hydrolyze the isothiuronium salt, thereby obtaining a polythiol compound represented by a specific Formula (5); and a process of adding hydrochloric acid at a concentration of from 25 to 36% to a solution containing the obtained polythiol compound and washing the solution at a temperature of from 10 to 50°C to purify the polythiol compound.
Patent Document 1: Japanese Patent Application Laid-Open (JP-A) No. H02-270859
Patent Document 2: WO 2014/027427

### SUMMARY OF INVENTION

### Technical Problem

A resin obtained by curing a polymerizable composition containing a polythiol compound may be required to have reduced yellow index and degree of opacity.

Actually, a polymerizable composition containing a polythiol compound often contains a compound other than the polythiol compound.

The present inventors have found that, in the case in which a polymerizable composition contains a compound other than the polythiol compound, the yellow index and degree of opacity tend to be easily impaired in the resulting resin.

As a result of various studies on the above tendency, the present inventors have found that it may be difficult to obtain a resin having reduced yellow index and degree of opacity.

As a resin obtained using a polythiol composition, for example, a thiourethane resin may be exemplified.

A thiourethane resin is usually produced using a polythiol composition and a polyisocyanate compound as raw materials.

There is a case in which further improvement of the heat resistance of a thiourethane resin is required.

An object of an embodiment of the present disclosure is to provide: a polythiol composition from which a resin having reduced yellow index and degree of opacity and being excellent in heat resistance can be produced; and applications thereof.

### Solution to Problem

Means to solve the above-described problems include the following embodiments.
<1> A polythiol composition, comprising:
   a polythiol compound (A) that is 4-mercaptomethyl-1,8-dimercapto-3,6-dithiaoctane; and
   a compound represented by the following Formula (1): 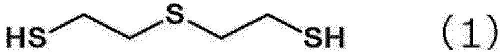
   wherein, in a high-performance liquid chromatography measurement, a peak area of the compound represented by the Formula (1) is 11.00 or less with respect to a total peak area 100 of compounds contained in the polythiol composition.
<2> The polythiol composition according to <1>, wherein, in the high-performance liquid chromatography measurement, the peak area of the compound represented by the Formula (1) is 13.00 or less with respect to a peak area 100 of the polythiol compound (A).
<3> The polythiol composition according to <2>, wherein the peak area of the compound represented by the Formula (1) is 8.00 or less with respect to the peak area 100 of the polythiol compound (A).
<4> The polythiol composition according to any one of <1> to <3>, further comprising a polythiol compound (B) comprising at least one selected from the group consisting of 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane.
<5> The polythiol composition according to <4>, wherein, in a high-performance liquid chromatography measurement, a peak area of the polythiol compound (B) is 3.10 or less with respect to a total peak area 100 of compounds contained in the polythiol composition.
<6> A polymerizable composition, comprising:
   the polythiol composition according to any one of <1> to <5>; and
   a polyiso(thio)cyanate compound.
<7> The polymerizable composition according to <6>, wherein the polyiso(thio)cyanate compound comprises at least one selected from the group consisting of pentamethylene diisocyanate, hexamethylene diisocyanate, xylylene diisocyanate, isophorone diisocyanate, bis(isocyanatomethyl)cyclohexane, bis(isocyanatocyclohexyl)methane, 2,5-bis(isocyanatomethyl)bicyclo-[2.2.1]-heptane, 2,6-bis(isocyanatomethyl)bicyclo-[2.2.1]-heptane, tolylene diisocyanate, 4,4'-diphenylmethane diisocyanate, and phenylene diisocyanate.
<8> The polymerizable composition according to <6>,
   wherein the polymerizable composition comprises a polyiso(thio)cyanate composition comprising the polyiso(thio)cyanate compound, and
   wherein the polyiso(thio)cyanate composition comprises: xylylene diisocyanate; and at least one selected from the group consisting of the following compound (N1), the following compound (N2), and the following compound (N3): 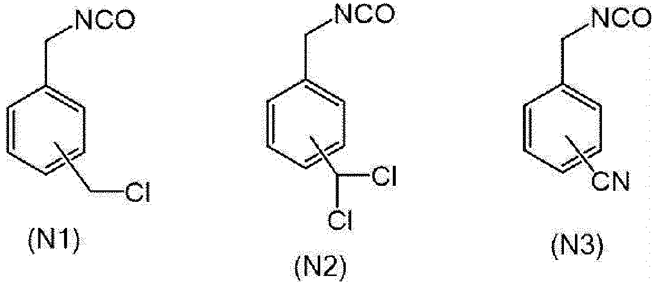 wherein:
      in a case in which the polyiso(thio)cyanate composition comprises the compound (N1), a peak area of the compound (N1) is 0.20 ppm or more with respect to a peak area 100 of xylylene diisocyanate in a gas chromatography measurement,
      in a case in which the polyiso(thio)cyanate composition comprises the compound (N2), a peak area of the compound (N2) is 0.05 ppm or more with respect to a peak area 100 of xylylene diisocyanate in a gas chromatography measurement, and
      in a case in which the polyiso(thio)cyanate composition comprises the compound (N3), a peak area of the compound (N3) is 0.10 ppm or more with respect to a peak area 100 of xylylene diisocyanate in a gas chromatography measurement.
<9> A resin, comprising a cured product of the polymerizable composition according to any one of <6> to <8>.
<10> A molded body, comprising the resin according to <9>.
<11> An optical material, comprising the resin according to <9>.
<12> A lens, comprising the resin according to <9>.

### Advantageous Effects of Invention

According to an embodiment of the present disclosure, a polythiol composition from which a resin having reduced yellow index and degree of opacity and being excellent in heat resistance can be produced; and applications thereof can be provided.

### DESCRIPTION OF EMBODIMENTS

In the disclosure, a numerical range indicated using "to" indicates a range including numerical values described before and after "to" as a minimum value and a maximum value, respectively.

With regard to the stepwise numerical ranges described herein, the upper limit value or the lower limit value described in a certain numerical range may be replaced with the upper limit value or the lower limit value of another stepwise numerical range or may be replaced with a value indicated in Examples.

Herein, the amount of each component in a material means the total amount of the plurality of substances present in the material, unless otherwise specified, when there is more than one substance corresponding to each component in the material.

Herein, "iso(thio)cyanate" means isocyanate or isothiocyanate.

### <<Polythiol Composition>>

A polythiol composition of the disclosure contains: a polythiol compound (A) that is 4-mercaptomethyl-1,8-dimercapto-3,6-dithiaoctane; and a compound represented by the following Formula (1), wherein, in a high-performance liquid chromatography measurement, the peak area of the compound represented by the Formula (1) is 11.00 or less with respect to the total peak area 100 of the compounds contained in the polythiol composition.

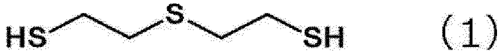

Since the polythiol composition of the disclosure contains the above-described configuration, a resin having reduced yellow index and degree of opacity and being excellent in heat resistance can be produced therefrom.

### (Polythiol Compound (A))

The polythiol composition of the disclosure contains a polythiol compound (A) that is 4-mercaptomethyl-1,8-dimercapto-3,6-dithiaoctane.

4-Mercaptomethyl-1,8-dimercapto-3,6-dithiaoctane is a compound represented by the following Formula (a-1):

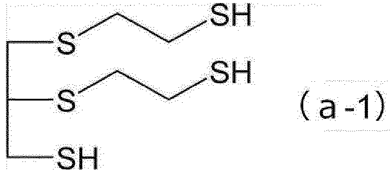

A method of producing the polythiol compound (A) is not particularly limited, and the polythiol compound (A) can be produced by a known method. For example, the polythiol compound (A) can be produced by the method described in WO 2014/027427. The polythiol compound (A) is preferably a compound obtained using a catalyst containing at least one selected from the group consisting of metal hydroxides such as sodium hydroxide and potassium hydroxide, and metal carbonates such as sodium carbonate and potassium carbonate, for example, on reacting 2-mercaptoethanol with an epihalohydrin compound.

In the polythiol composition of the disclosure, in a high-performance liquid chromatography measurement, the peak area of the polythiol compound (A) is preferably 80.00 or more with respect to the total peak area 100 of the compounds contained in the polythiol composition.

When the peak area of the polythiol compound (A) is 80.00 or more, formability of the thiourethane resin is excellent.

From the above viewpoint, the peak area of the polythiol compound (A) is preferably 81.00 or more, more preferably 82.00 or more, and still more preferably 86.50 or more with respect to the total peak area 100 of the compounds contained in the polythiol composition.

The "peak area of the polythiol compound (A) with respect to the total peak area 100 of the compounds contained in the polythiol composition" means a relative value of the peak area of the polythiol compound (A) in the case in which the total peak area of the compounds contained in the polythiol composition is taken as 100.

In the high-performance liquid chromatography measurement, the peak area of the polythiol compound (A) is preferably less than 100, more preferably 99.50 or less, still more preferably 95.00 or less, and particularly preferably 89.50 or less, with respect to the total peak area 100 of the compounds contained in the polythiol composition, from the viewpoint of producing a thiourethane resin excellent in heat resistance.

### <Measurement of Peak Area of Polythiol Compound (A)>

The peak area of the polythiol compound (A) can be determined by performing a high-performance liquid chromatography (HPLC) measurement under the following conditions.

The peak area appearing at a retention time of 11.0 minutes to 13.5 minutes may be regarded as the peak area of the polythiol compound (A), and the ratio thereof with respect to the total peak area 100 of the compounds contained in the polythiol composition may be calculated.

### (HPLC Conditions)

Column: YMC-Pack ODS-A A-312 (S5Φ6 mm × 150 mm)
Mobile phase: acetonitrile / 0.01 mol/L-potassium dihydrogen phosphate aqueous solution = 60/40 (vol/vol)
Column temperature: 40°C
Flow rate: 1.0 ml/min
Detector: UV detector, wavelength 230 nm
Preparation of measurement solution: 160 mg of a polythiol composition is dissolved and mixed with 10 ml of acetonitrile.
Injection amount: 2 µL

### <Compound Represented by Formula (1)>

The polythiol composition of the disclosure contains a compound represented by the following Formula (1).

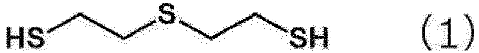

In the polythiol composition of the disclosure, in a high-performance liquid chromatography measurement, the peak area of the compound represented by the Formula (1) is 11.00 or less with respect to the total peak area 100 of the compounds contained in the polythiol composition.

Since the peak area of the compound represented by the Formula (1) is 11.00 or less, a resin having reduced yellow index and degree of opacity and being excellent in heat resistance can be produced.

From the above viewpoint, the peak area of the compound represented by the Formula (1) is preferably 8.50 or less, more preferably 6.00 or less, and still more preferably 4.00 or less, with respect to the total peak area 100 of the compounds contained in the polythiol composition.

In the high-performance liquid chromatography measurement, the peak area of the compound represented by the Formula (1) is preferably more than 0, more preferably 0.01 or more, still more preferably 0.10 or more, and particularly preferably 0.27 or more, with respect to the total peak area 100 of the compounds contained in the polythiol composition, from the viewpoint of reducing the burden of work of purifying and removing the compound represented by the Formula (1) from the polythiol composition.

In the polythiol composition of the disclosure, in the high-performance liquid chromatography measurement, the peak area of the compound represented by the Formula (1) is 13.00 or less with respect to the peak area 100 of the polythiol compound (A).

When the peak area of the compound represented by the Formula (1) is 13.00 or less, a resin having reduced yellow index and degree of opacity and being excellent in heat resistance can be produced.

From the above viewpoint, the peak area of the compound represented by the Formula (1) is preferably 12.00 or less, more preferably 8.00 or less, and still more preferably 4.50 or less, with respect to the peak area 100 of the polythiol compound (A).

In the high-performance liquid chromatography measurement, the peak area of the compound represented by the Formula (1) is preferably more than 0, more preferably 0.01 or more, still more preferably 0.10 or more, and particularly preferably 0.30 or more, with respect to the peak area 100 of the polythiol compound (A), from the viewpoint of reducing the burden of work of purifying and removing the compound represented by the Formula (1) from the polythiol composition.

### <Measurement of Peak Area of Compound Represented by Formula (1)>

The peak area of the compound represented by the Formula (1) can be determined by performing a high-performance liquid chromatography (HPLC) measurement under the following conditions.

The peak area appearing at a retention time of 6.2 minutes to 7.0 minutes may be regarded as the peak area of the compound represented by the Formula (1), and the ratio thereof with respect to the total peak area 100 of the compounds contained in the polythiol composition or the peak area 100 of the polythiol compound (A) may be calculated.

### (HPLC Conditions)

Column: YMC-Pack ODS-A A-312 (S5Φ6 mm × 150 mm)
Mobile phase: acetonitrile / 0.01 mol/L-potassium dihydrogen phosphate aqueous solution = 60/40 (vol/vol)
Column temperature: 40°C
Flow rate: 1.0 ml/min
Detector: UV detector, wavelength 230 nm
Preparation of measurement solution: 160 mg of a polythiol composition is dissolved and mixed with 10 ml of acetonitrile.
Injection amount: 2 µL

### <Compound Other Than Polythiol Compound (A) and Compound Represented by Formula (1)>

The polythiol composition of the disclosure may contain a compound other than the polythiol compound (A) and the compound represented by the Formula (1).

For example, the polythiol composition of the disclosure may contain a polythiol compound other than the polythiol compound (A) and the compound represented by the Formula (1) (hereinafter, also referred to as "another polythiol compound").

Examples of another polythiol compound include methanedithiol, 1,2-ethanedithiol, 1,2,3-propanetrithiol, pentaerythritol tetrakis(2-mercaptoacetate), pentaerythritol tetrakis(3-mercaptopropionate), tetrakis(mercaptomethylthiomethyl)methane, tetrakis(2-mercaptoethylthiomethyl)methane, tetrakis(3-mercaptopropylthiomethyl)methane, bis(2-mercaptoethyl)sulfide, bis(2,3-dimercaptopropyl)sulfide, 4,8-dimercaptomethyl-1,11-dimercapto-3,6,9-trithiaundecane, 4,7-dimercaptomethyl-1,11-dimercapto-3,6,9-trithiaundecane, 5,7-dimercaptomethyl-1,11-dimercapto-3,6,9-trithiaundecane, 2,5-dimercaptomethyl-1,4-dithiane, 2,5-dimercapto-1,4-dithiane, 2,5-dimercaptomethyl-2,5-dimethyl-1,4-dithiane, 1,1,3,3-tetrakis(mercaptomethylthio)propane, 1,1,2,2-tetrakis(mercaptomethylthio)ethane, and 4,6-bis(mercaptomethylthio)-1,3-dithiane.

### (Polythiol Compound (B))

The polythiol composition of the disclosure preferably contains a polythiol compound (B) comprising at least one selected from the group consisting of 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane.

In a high-performance liquid chromatography measurement, the peak area of the polythiol compound (B) is preferably 3.10 or less with respect to the total peak area 100 of the compounds contained in the polythiol composition.

Further, the peak area of the polythiol compound (B) is more preferably 3.05 or less with respect to the total peak area 100 of the compounds contained in the polythiol composition.

In the high-performance liquid chromatography measurement, the peak area of the polythiol compound (B) may be more than 0, may be 0.10 or more, or may be 0.20 or more, with respect to the total peak area 100 of the compounds contained in the polythiol composition.

### <Measurement of Peak Area of Polythiol Compound (B)>

The peak area of the polythiol compound (B) with respect to the total peak area 100 of the compounds contained in the polythiol composition can be determined by performing a high-performance liquid chromatography (HPLC) measurement under the following conditions.

The peak area appearing at a retention time of 21.0 minutes to 24.0 minutes may be regarded as the peak area of the polythiol compound (B), and the ratio thereof with respect to the total peak area 100 of the compounds contained in the polythiol composition may be calculated.

### (HPLC Conditions)

Column: YMC-Pack ODS-A A-312 (S5Φ6 mm × 150 mm)
Mobile phase: acetonitrile / 0.01 mol/L-potassium dihydrogen phosphate aqueous solution = 60/40 (vol/vol)
Column temperature: 40°C
Flow rate: 1.0 ml/min
Detector: UV detector, wavelength 230 nm
Preparation of measurement solution: 160 mg of a polythiol composition is dissolved and mixed with 10 ml of acetonitrile.
Injection amount: 2 µL

### <Compound represented by Formula (X1)>

As a compound that may be contained in the polythiol composition of the disclosure and that is other than the polythiol compound (A) and the compound represented by the Formula (1), a compound represented by the following Formula (X1) may be exemplified.

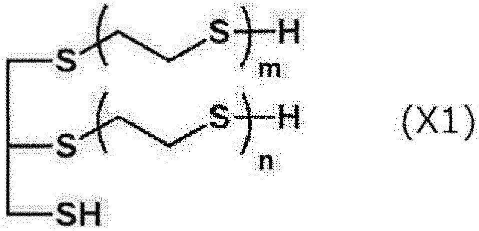

In the Formula (X1), m and n each independently represent 0 or 1, and m + n = 1.

The structure of the compound represented by the Formula (X1) is similar to the structure of the polythiol compound (A) in the disclosure (that is, 4-mercaptomethyl-1,8-dimercapto-3,6-dithiaoctane, the compound represented by the Formula (a-1)).

The structure of the compound represented by the Formula (X1) differs from the structure of the compound represented by the Formula (a-1) in that, in the former, m and n each independently represent 0 or 1, and m + n = 1, whereas, in the latter, m and n each represent 1 (that is, m + n = 2).

Examples of the compound represented by the Formula (X1) include a compound represented by Formula (X1a) and a compound represented by Formula (X1b).

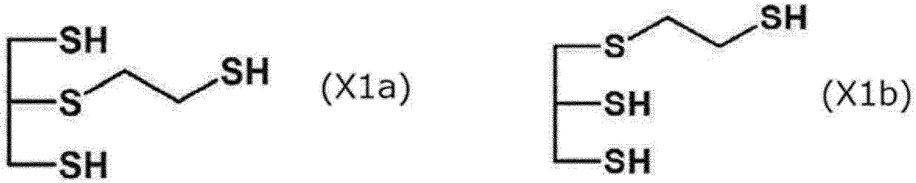

The polythiol composition of the disclosure may also contain plural compounds represented by the Formula (X1). For example, the polythiol composition of the disclosure may contain a mixture of a compound represented by the Formula (X1a) and a compound represented by the Formula (X1b) as a compound represented by the Formula (X1).

The compound represented by the Formula (X1) may be a compound represented by the Formula (X1a), a compound represented by the Formula (X1b), or a mixture of a compound represented by the Formula (X1a) and a compound represented by the Formula (X1b).

When the compound represented by the Formula (X1) includes a compound represented by the Formula (X1a) and a compound represented by the Formula (X1b), the content of the compound represented by the Formula (X1a) is preferably 80% by mass or more, more preferably 90% by mass or more, and still more preferably 95% by mass or more, with respect to the total content of the compound represented by the Formula (X1a) and the compound represented by the Formula (X1b).

### [Compound (XB)]

As a compound other than the polythiol compound (A) and the compound (B1), the following compound (XB) may also be exemplified.

The compound (XB) may be a compound obtained by replacing at least one of three or more mercapto groups in a polythiol compound (hereinafter, also referred to as a "polythiol compound (XA)") containing three or more mercapto groups with a group represented by the following Formula (XB-1).

Here, the scope of the polythiol compound (that is, the polythiol compound (XA)) containing three or more mercapto groups includes the polythiol compound (A) in the disclosure (that is, 4-mercaptomethyl-1,8-dimercapto-3,6-dithiaoctane, the compound represented by the Formula (a-1)).

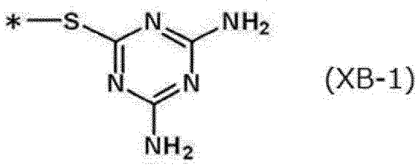

In the Formula (XB-1), * represents a bonding position.

Examples of the compound (XB) are shown below, but the compound (XB) is not limited to the following examples.

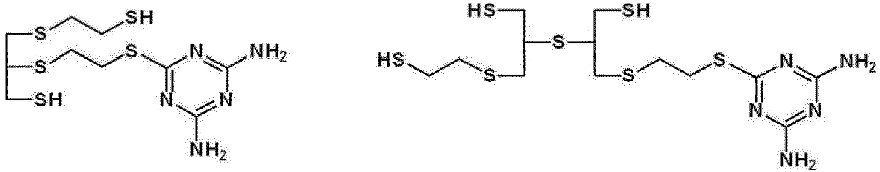

In the case in which the polythiol composition of the disclosure contains the compound represented by the Formula (X1) and the compound (XB), the ratio thereof (the compound represented by the Formula (X1) / the compound (XB)) is preferably from 1 to 30, more preferably from 3 to 24, and still more preferably from 5 to 18, from the viewpoint of reducing the yellow index and degree of opacity of a resin to be obtained and from the viewpoint of favorably maintaining the pot life of the polymerizable composition of the disclosure.

### <<Polymerizable Composition>>

The polymerizable composition of the disclosure contains the polythiol composition of the disclosure and a polyiso(thio)cyanate compound.

### (Polyiso(thio)cyanate Compound)

The polyiso(thio)cyanate compound is not particularly limited as long as the effects of the disclosure can be exerted, and any conventionally known compound can be used. Any compound having at least two iso(thio)cyanate groups in one molecule may be used which is not particularly limited, and specific examples thereof include:
aliphatic polyisocyanate compounds such as tetramethylene diisocyanate, pentamethylene diisocyanate, hexamethylene diisocyanate, heptamethylene diisocyanate, octamethylene diisocyanate, 2,2,4-trimethylhexamethylene diisocyanate, 2,4,4-trimethylhexamethylene diisocyanate, lysine diisocyanate methyl ester, lysine triisocyanate, and xylylene diisocyanate;
alicyclic polyisocyanate compounds such as isophorone diisocyanate, bis(isocyanatomethyl)cyclohexane, bis(isocyanatocyclohexyl)methane, dicyclohexyldimethylmethane diisocyanate, 2,5-bis(isocyanatomethyl)bicyclo-[2.2.1]-heptane, 2,6-bis(isocyanatomethyl)bicyclo-[2.2.1]-heptane, 3,8-bis(isocyanatomethyl)tricyclodecane, 3,9-bis(isocyanatomethyl)tricyclodecane, 4,8-bis(isocyanatomethyl)tricyclodecane, and 4,9-bis(isocyanatomethyl)tricyclodecane;
aromatic polyisocyanate compounds such as tolylene diisocyanate, 4,4'-diphenylmethane diisocyanate, diphenyl sulfide-4,4'-diisocyanate, and phenylene diisocyanate;
heterocyclic polyisocyanate compounds such as 2,5-diisocyanatothiophene, 2,5-bis(isocyanatomethyl)thiophene, 2,5-diisocyanatotetrahydrothiophene, 2,5-bis(isocyanatomethyl)tetrahydrothiophene, 3,4-bis(isocyanatomethyl)tetrahydrothiophene, 2,5-diisocyanato-1,4-dithiane, 2,5-bis(isocyanatomethyl)-1,4-dithiane, 4,5-diisocyanato-1,3-dithiolane, and 4,5-bis(isocyanatomethyl)-1,3-dithiolane;
aliphatic polyisothiocyanate compounds such as hexamethylene diisothiocyanate, lysine diisothiocyanate methyl ester, lysine triisothiocyanate, and xylylene diisothiocyanate;
alicyclic polyisothiocyanate compounds such as isophorone diisothiocyanate, bis(isothiocyanatomethyl)cyclohexane, bis(isothiocyanatocyclohexyl)methane, cyclohexane diisothiocyanate, methylcyclohexane diisothiocyanate, 2,5-bis(isothiocyanatomethyl)bicyclo-[2.2.1]-heptane, 2,6-bis(isothiocyanatomethyl)bicyclo-[2.2.1]-heptane, 3,8-bis(isothiocyanatomethyl)tricyclodecane, 3,9-bis(isothiocyanatomethyl)tricyclodecane, 4,8-bis(isothiocyanatomethyl)tricyclodecane, and 4,9-bis(isothiocyanatomethyl)tricyclodecane;
aromatic polyisothiocyanate compounds such as tolylene diisothiocyanate, 4,4'-diphenylmethane diisothiocyanate, and diphenyl disulfide-4,4'-diisothiocyanate; and
sulfur-containing heterocyclic polyisothiocyanate compounds such as 2,5-diisothiocyanatothiophene, 2,5-bis(isothiocyanatomethyl)thiophene, 2,5-isothiocyanatotetrahydrothiophene, 2,5-bis(isothiocyanatomethyl)tetrahydrothiophene, 3,4-bis(isothiocyanatomethyl)tetrahydrothiophene, 2,5-diisothiocyanato-1,4-dithiane, 2,5-bis(isothiocyanatomethyl)-1,4-dithiane, 4,5-diisothiocyanato-1,3-dithiolane, and 4,5-bis(isothiocyanatomethyl)-1,3-dithiolane.

The polyiso(thio)cyanate compound can contain at least one selected from these compounds.

As the polyiso(thio)cyanate compound, a halogen-substituted product such as a chlorine-substituted product or a bromine-substituted product, an alkyl-substituted product, an alkoxy-substituted product, a nitro-substituted product, a prepolymer-type modified product with a polyhydric alcohol, a carbodiimide-modified product, a urea-modified product, a burette-modified product, or a dimerization or trimerization reaction product of these compounds can also be used.

The polyiso(thio)cyanate compound is preferably a polyisocyanate compound, and
preferably comprises at least one selected from the group consisting of pentamethylene diisocyanate, hexamethylene diisocyanate, xylylene diisocyanate, isophorone diisocyanate, bis(isocyanatomethyl)cyclohexane, bis(isocyanatocyclohexyl)methane, 2,5-bis(isocyanatomethyl)bicyclo-[2.2.1]-heptane, 2,6-bis(isocyanatomethyl)bicyclo-[2.2.1]-heptane, tolylene diisocyanate, 4,4'-diphenylmethane diisocyanate, and phenylene diisocyanate.

The mixing ratio of the polythiol composition and the polyiso(thio)cyanate compound is not particularly limited, and for example, the molar ratio of mercapto groups of the polythiol compound contained in the polythiol composition to iso(thio)cyanate groups of the polyiso(thio)cyanate compound (mercapto groups/iso(thio)cyanate groups) is preferably from 0.5 to 3.0, more preferably from 0.6 to 2.0, and still more preferably from 0.8 to 1.3. In the case in which the mixing ratio is within the above range, it tends to be possible to satisfy various performance characteristics such as a refractive index and heat resistance required for a plastic lens or the like in a well-balanced manner.

The polymerizable composition of the disclosure may contain a polyiso(thio)cyanate composition containing the polyiso(thio)cyanate compound.

Herein, a polyiso(thio)cyanate composition means a composition containing at least one polyiso(thio)cyanate compound.

The polyiso(thio)cyanate composition may contain, as an impurity, a component other than a polyiso(thio)cyanate compound.

The polyiso(thio)cyanate composition preferably contains, as a main component, at least one polyiso(thio)cyanate compound.

The polyiso(thio)cyanate composition preferably contains xylylene diisocyanate.

Hereinafter, a polyiso(thio)cyanate composition containing xylylene diisocyanate is also referred to as an XDI composition.

The XDI composition preferably contains xylylene diisocyanate as a main component.

The XDI composition preferably contains at least one selected from the group consisting of the following compound (N1), the following compound (N2), and the following compound (N3).

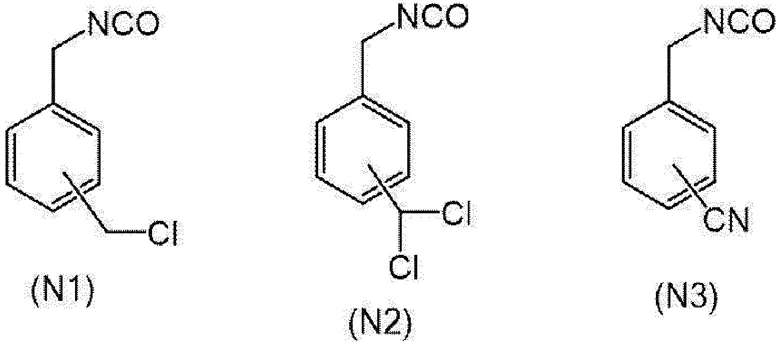

Hereinafter, preferable aspects of the XDI composition are shown from the viewpoints of stability of the polyiso(thio)cyanate composition and more excellent transparency of a resin formed using the polyiso(thio)cyanate composition.

In a case in which the XDI composition contains the compound (N1), a peak area of the compound (N1) is preferably 0.20 ppm or more with respect to a peak area 1 of xylylene diisocyanate in a gas chromatography measurement under the following GC condition 1.

### - GC condition 1 -

Packing material; DB-1 (film thickness) 1.5 µm
Column; internal diameter 0.53 mm × length 60 m (manufactured by Agilent Technologies, Inc.)
Oven temperature; increased at 3°C/min from 130°C to 220°C, and increased at 10°C/min to 300°C after reaching 220°C
Split ratio; pulsed splitless method
Inlet temperature; 280°C
Detector temperature; 300°C
Carrier gas; N₂ 158 kPa, H₂ 55 kPa, Air 45 kPa (constant pressure control)
Solvent; chloroform
Sample concentration: 2.0 mass% chloroform solution
Injection amount; 2 µL
Detection method; FID

The peak area of the compound (N1) is more preferably 5.0 ppm or more, still more preferably 50 ppm or more, and still more preferably 100 ppm or more with respect to the peak area 1 of xylylene diisocyanate.

The peak area of the compound (N1) is preferably 4000 ppm or less, more preferably 3000 ppm or less, still more preferably 2000 ppm or less, still more preferably 1500 ppm or less, and still more preferably 1000 ppm or less with respect to the peak area 1 of xylylene diisocyanate.

The peak area of the compound (N1) can be measured in accordance with the method described in paragraph [0377] of Japanese patent No. 6373536.

In a case in which the XDI composition contains the compound (N2), a peak area of the compound (N2) is preferably 0.05 ppm or more with respect to a peak area 1 of xylylene diisocyanate in a gas chromatography measurement under the following GC condition 2.

### - GC condition 2 -

Column; HP-50+, internal diameter 0.25 mm × length 30 m × film thickness 0.25 µm
(manufactured by Hewlett-Packard Company)
Oven temperature; increased at 10°C/min from 50°C to 280°C, and held for 6 min after reaching 280°C
Split ratio; pulsed splitless method
Inlet temperature; 200°C
Detector temperature; 280°C
Carrier gas; He
Carrier gas flow rate; 1.0 ml/min (constant flow rate control)
Sample concentration: 1.0 mass% dichloromethane solution
Injection amount; 1.0 µL
Detection method; SIM (monitoring ion: m/z 180, 215) (Xylylene diisocyanate (XDI) content ratio)

The peak area of the compound (N2) is more preferably 0.1 ppm or more, still more preferably 0.3 ppm or more, and still more preferably 0.6 ppm or more with respect to the peak area 1 of xylylene diisocyanate.

The peak area of the compound (N2) is preferably 200 ppm or less, more preferably 150 ppm or less, still more preferably 100 ppm or less, still more preferably 80 ppm or less, still more preferably 70 ppm or less, and still more preferably 60 ppm or less with respect to the peak area 1 of xylylene diisocyanate.

The peak area of the compound (N2) can be measured in accordance with the method described in paragraphs [0375] and [0376] of Japanese patent No. 6373536.

In a case in which the XDI composition contains the compound (N3), a peak area of the compound (N3) is preferably 0.10 ppm or more with respect to a peak area 1 of xylylene diisocyanate in a gas chromatography measurement under the above GC condition 1.

The peak area of the compound (N3) is more preferably 0.1 ppm or more, still more preferably 3.0 ppm or more, and still more preferably 5.0 ppm or more with respect to the peak area 1 of xylylene diisocyanate.

The peak area of the compound (N3) is preferably 1000 ppm or less, more preferably 500 ppm or less, still more preferably 300 ppm or less, still more preferably 100 ppm or less, and still more preferably 75 ppm or less with respect to the peak area 1 of xylylene diisocyanate.

The peak area of the compound (N3) can be measured in accordance with the method described in paragraph [0377] of Japanese patent No. 6373536.

The acid content in the XDI composition is preferably 3000 ppm or less, more preferably 2000 ppm or less, still more preferably 1000 ppm or less, still more preferably 100 ppm or less, still more preferably 50 ppm or less, still more preferably 30 ppm or less, and still more preferably less than 15 ppm.

The lower limit value of the acid content in the XDI composition is not particularly limited, and the lower limit value is, for example, 1 ppm.

The acid content in the XDI composition can be measured in accordance with the method described in paragraph [0091] of WO 2021/256417.

Further, the XDI composition may contain a stabilizing agent.

The polymerizable composition of the disclosure may contain other components other than the polythiol compound and the polyiso(thio)cyanate compound for the purpose of improving various physical properties and operability of the resin, polymerization reactivity of the polymerizable composition, and the like.

Examples of the other components include a polymerization catalyst, an internal mold release agent, a resin modifier, a chain extender, a crosslinking agent, a radical scavenger, a light stabilizer, an ultraviolet absorber, an antioxidant, an oil soluble dye, a filler, an adhesion improver, an antibacterial agent, an antistatic agent, a dye, a fluorescent brightener, a fluorescent pigment, and a blue ink agent such as an inorganic pigment.

Examples of the polymerization catalyst include a tertiary amine compound, an inorganic acid salt or organic acid salt thereof, a metal compound, a quaternary ammonium salt, and an organic sulfonic acid.

As the internal mold release agent, an acidic phosphate ester can be used. Examples of the acidic phosphate ester include a phosphoric acid monoester and a phosphoric acid diester, which can be used singly or in a mixture of two or more kinds thereof.

Examples of the resin modifier include an episulfide compound, an alcohol compound, an amine compound, an epoxy compound, an organic acid and an anhydride thereof, and an olefin compound including a (meth)acrylate compound.

The polymerizable composition of the disclosure can be obtained by mixing the above components.

### <<Molded Body>>

The molded body of the disclosure contains the resin of the disclosure.

The resin of the disclosure contains a cured product of the polymerizable composition of the disclosure.

The method of producing the molded body of the disclosure is not particularly limited, and examples of a preferred production method include cast polymerization. First, a polymerizable composition is poured between molding molds held with a gasket, tape, or the like. At this time, depending on physical properties required for a plastic lens to be obtained, it is often preferable to perform defoaming treatment under reduced pressure, filtration treatment such as filtration under pressure or reduced pressure, or the like if necessary.

The polymerization conditions are not limited because the conditions greatly vary depending on the composition of the polymerizable composition, the type and amount of the catalyst to be used, the shape of the mold, and the like. For example, the polymerization is performed at a temperature of from -50°C to 150°C over from 1 hour to 50 hours. In some cases, it is preferable to cure the composition in from 1 hour to 48 hours while the temperature is maintained or gradually raised in a temperature range of 10°C to 150°C.

The molded body may be subjected to a treatment such as annealing if necessary. The treatment such as annealing is usually performed at from 50°C to 150°C, preferably at from 90°C to 140°C, and more preferably at from 100°C to 130°C.

### [Applications]

The resin to be obtained from the polymerizable composition of the disclosure can be used as a material for producing molded bodies of various shapes by changing the type of mold during cast polymerization.

### <<Optical Material>>

The optical material of the disclosure contains the resin of the disclosure.

From a molded body obtained from the polymerizable composition of the disclosure, it is possible to obtain a material having a reduced yellow index without impairing transparency. From a molded body obtained from the polymerizable composition containing the polythiol composition of the first embodiment, it is also possible to obtain a material having excellent degree of opacity.

Thus, such materials can be used for various optical materials such as plastic lenses.

### <<Lens>>

The lens of the disclosure contains the resin of the disclosure.

As the optical material, a lens is particularly suitable.

Examples of the lens include a plastic spectacle lens and a plastic polarizing lens.

### [Plastic Spectacle Lens]

The plastic spectacle lens including a lens substrate formed of the molded body of the disclosure may be provided with a coating layer on one side or both sides if necessary.

The plastic spectacle lens of the disclosure comprises a lens substrate including a cured product of the polymerizable composition described above, and a coating layer.

Specific examples of the coating layer include a primer layer, a hard coat layer, an antireflection layer, an antifogging coat layer, an antifouling layer, and a water-repellent layer. These coating layers each can be used singly, or a plurality of coating layers can be layered for use. In the case in which the coating layer is applied to both sides, the same coating layer or a different coating layer may be applied to each side.

In each of these coating layers, known additives such as an infrared absorber for the purpose of protecting eyes from infrared rays, a light stabilizer or an antioxidant for the purpose of improving the weather resistance of the lens, a photochromic compound, a dye, or a pigment for the purpose of improving the fashion of the lens, and additionally, an antistatic agent and the like for the purpose of improving the performance of the lens may be used in combination.

For the layer to be coated by coating, various leveling agents for the purpose of improving coatability may be used.

An anti-fog layer, an anti-contamination layer, or a water-repellent layer may be formed on the antireflection layer if necessary.

Although the embodiments of the disclosure have been described hereinabove, these are exemplary for the disclosure, and various configurations other than those described above can be adopted as long as the effects of the disclosure are not impaired.

### EXAMPLES

Hereinafter, the disclosure will be described in detail with reference to Examples. The disclosure is not limited to the description of these Examples in any way. Unless otherwise specified, "parts" are on a mass basis.

### <Evaluation Method>

In Examples, methods for evaluating the respective physical properties of plastic lenses are as follows. The results are as shown in Table 1.
- Yellow Index (also referred to as YI)

A resin was prepared as a circular plastic flat plate having a thickness of 9 mm and a diameter of 75 mm, and the YI value was determined using a spectrophotometer CR-400 manufactured by Konica Minolta, Inc.

There is a correlation between the YI value and the yellow index, in which a smaller YI value leads to a lower yellow index of the plastic flat plate and a larger YI value leads to a higher yellow index.
- Degree of opacity

A resin was prepared as a circular plastic flat plate having a thickness of 9 mm and a diameter of 75 mm, and light from a light source (LUMINAR ACE LA-150A manufactured by HAYASHI-REPIC CO., LTD.) was allowed to transmit through the flat plate from a side thereof. An image of light from the front of the flat plate was captured in an image processing apparatus (manufactured by Ube Information Systems, Inc.), and gradation processing was performed on the captured image. The degree of gradation of the processed image was quantified on a pixel to pixel basis, the average value of the numerical values of the degree of gradation of each pixel was obtained, and the degree of opacity of the flat plate was determined.

With a smaller degree of opacity, the degree of impairing the transparency of the resin (here, the flat plate) is smaller (i.e., the resin has excellent transparency).
- Heat resistance

A resin test piece having a length of 10 mm, a width of 10 mm, and a thickness of 2.5 mm was prepared, and a thermomechanical analyzer TMA-60 manufactured by Shimadzu Corporation was used to measure a glass transition temperature (Tg) based on a TMA penetration method (load: 50 g, pin tip: 0.5 mmφ, temperature rising rate: 10°C/min), which was used as an index of heat resistance.

With a higher glass transition temperature (Tg), the resin is excellent in heat resistance.

### <Preparation of Polythiol Composition (A)>

Into a reactor, charged were 124.6 parts by mass of 2-mercaptoethanol and 18.3 parts by mass of degassed water. Thereinto, at from 12°C to 35°C, 101.5 parts by mass of a 32 mass% concentration sodium hydroxide aqueous solution were added dropwise over 40 minutes, then 73.6 parts by mass of epichlorohydrin were added dropwise over 4.5 hours at from 29°C to 36°C, and stirring was continued for 40 minutes. It was confirmed from the NMR data of the obtained reaction liquid that 1,3-bis(2-hydroxyethylthio)-2-propanol was generated.

Next, into the reactor including 1,3-bis(2-hydroxyethylthio)-2-propanol, 331.5 parts by mass of 35.5 mass% concentration hydrochloric acid were charged, then 183.8 parts by mass of thiourea having a purity of 99.90 mass% were charged, and a thiuronium salt-forming reaction was performed by stirring the resulting mixture at 110°C under reflux for 3 hours.

After cooling the reaction liquid after the thiuronium salt-forming reaction to 45°C, 320.5 parts by mass of toluene were added thereto, the mixture was cooled to 31°C, 243.1 parts by mass of a 25 mass% aqueous ammonia solution were charged at from 31°C to 41°C over 44 minutes. The mixture was subjected to a hydrolysis reaction by stirring at from 54°C to 62°C for 3 hours to obtain a toluene solution of a polythiol containing 4-mercaptomethyl-1,8-dimercapto-3,6-dithiaoctane as a main component.

To this toluene solution, 162.8 parts by mass of 35.5 mass% concentration hydrochloric acid were added, and the toluene solution was acid-washed at from 35°C to 43°C for 1 hour. To the toluene solution after the acid-washing, a procedure of addition of 174.1 parts by mass of degassed water and washing at from 35°C to 45°C for 30 minutes was performed twice. To the toluene solution after the degassed water washing, 162.1 parts by mass of 0.1 mass% concentration ammonia water were added, and the toluene solution was washed for 30 minutes. To the toluene solution after the ammonia water washing, a procedure of addition of 174.2 parts by mass of degassed water and washing at from 35°C to 45°C for 30 minutes was performed twice.

Next, from the toluene solution after the degassed water washing, toluene and a trace amount of moisture were removed under heating and reduced pressure, and then the residue was filtered under reduced pressure with a 1.2 µm PTFE-type membrane filter to obtain 205.0 parts by mass of a polythiol composition (A) containing, as a main component, 4-mercaptomethyl-1,8-dimercapto-3,6-dithiaoctane that was the polythiol compound (A).

### <Preparation of Polythiol Composition (B)>

Into a reactor, charged were 51.2 parts by mass of 2-mercaptoethanol, 26.5 parts by mass of degassed water, and 0.16 parts by mass of a 49 mass% concentration sodium hydroxide aqueous solution. Thereinto, 61.99 parts by mass of epichlorohydrin were added dropwise over 6.5 hours at from 9°C to 11°C, and stirring was continued for 60 minutes. It was confirmed from the NMR data of the obtained reaction liquid that 1-chloro-3-(2-hydroxyethylthio)-2-propanol was generated.

Next, into the reactor including 1-chloro-3-(2-hydroxyethylthio)-2-propanol, 150.0 parts by mass of a 17.3 mass% concentration sodium sulfide aqueous solution were added dropwise over 5.5 hours at from 7°C to 37°C, and stirring was performed for 120 minutes. As a result, it was confirmed from the NMR data that 1,5,9,13-tetrahydroxy-3,7,11-trithiatridecane was generated.

Next, into the reactor including 1,5,9,13-tetrahydroxy-3,7,11-trithiatridecane, 279.0 parts by mass of 35.5 mass% concentration hydrochloric acid were charged, then 125.8 parts by mass of thiourea having a purity of 99.90% were charged, and a thiuronium salt-forming reaction was performed by stirring the resulting mixture at 110°C under reflux for 3 hours.

After cooling the reaction liquid after the thiuronium salt-forming reaction to 45°C, 214.0 parts by mass of toluene were added, the mixture was cooled to 26°C, 206.2 parts by mass of a 25 mass% concentration aqueous ammonia solution were charged thereinto at from 26°C to 50°C over 30 minutes. The mixture was subjected to a hydrolysis reaction by stirring at from 50°C to 65°C for 1 hour to obtain a toluene solution of a polythiol containing, as a main component, at least one selected from the group consisting of 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane.

To the obtained toluene solution, a procedure of addition of 59.4 parts by mass of 36 mass% concentration hydrochloric acid and acid-washing at from 34°C to 39°C for 30 minutes was performed twice. To the toluene solution after the acid-washing, a procedure of addition of 118.7 parts by mass of degassed water and washing at from 35°C to 45°C for 30 minutes was performed five times.

Next, from the toluene solution after the degassed water washing, toluene and a trace amount of moisture were removed under heating and reduced pressure, and then the residue was filtered under reduced pressure with a 1.2 µm PTFE-type membrane filter to obtain 115.9 parts by mass of a polythiol composition (B) containing, as a main component, at least one selected from the group consisting of 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane.

### <Preparation of Polythiol Composition (C)>

The polythiol composition (A) and the polythiol composition (B) were mixed at arbitrary ratio to prepare a polythiol composition (C).

### [Example 1 to Example 6]

The polythiol composition (C) obtained as described above and a compound represented by the Formula (1) were mixed to obtain a polythiol composition.

At this time, each mixing was performed while changing the mixing ratio so that the peak area of the compound represented by the Formula (1) with respect to the total peak area 100 of the compounds contained in the polythiol composition was the value described in Table 1.

### <Measurement of proportions (area%) of polythiol compound (A), polythiol compound (B), and compound represented by Formula (1)>

The proportions (area%) of the polythiol compound (A), the polythiol compound (B), and the compound represented by the Formula (1) by HPLC were measured by the methods described in the sections of <Measurement of Peak Area of Polythiol Compound (A)>, <Measurement of Peak Area of Polythiol Compound (B)>, and <Measurement of Peak Area of Compound Represented by Formula (1)> described above, respectively.

The results are shown in Table 1.

### <Production of Plastic Lens>

### [Production Example 1]

52 parts by mass of m-xylylene diisocyanate, 0.015 parts by mass of dibutyltin dichloride as a curing catalyst, 0.10 parts by mass of ZELEC UN (trade name, a product manufactured by Stepan Company; acidic phosphate ester), and 0.05 parts by mass of VIOSORB 583 (manufactured by KYODO CHEMICAL CO., LTD.; ultraviolet absorber) were mixed and dissolved at 20°C. Thereinto, 48 parts by mass of the polythiol composition of Example 1 were charged and mixed to obtain a mixed homogeneous liquid. After this homogeneous liquid was degassed at 600 Pa for 1 hour and filtered with a 1 µm TEFLON (R) filter, the filtered liquid was poured into a mold die composed of a glass mold and tape. The mold die was placed into an oven, gradually heated up from 10°C to 120°C, and polymerization was performed for 38 hours. After the polymerization was finished, the mold die was taken out from the oven to obtain a resin by releasing from the mold die. The obtained resin was further annealed at 120°C for 1 hour to produce a plastic lens. Physical properties were each determined based on each of the methods of evaluating physical properties of the plastic lens described above.

### [Production Example 2]

A plastic lens was produced by the same method as that described in Production Example 1 except that 48 parts by mass of the polythiol composition of Example 1 were replaced by 48 parts by mass of the polythiol composition of Example 2 in Production Example 1. Physical properties were each determined based on each of the methods of evaluating physical properties of the plastic lens described above.

### [Production Example 3]

A plastic lens was produced by the same method as that described in Production Example 1 except that 48 parts by mass of the polythiol composition of Example 1 were replaced by 48 parts by mass of the polythiol composition of Example 3 in Production Example 1. Physical properties were each determined based on each of the methods of evaluating physical properties of the plastic lens described above.

### [Production Example 4]

A plastic lens was produced by the same method as that described in Production Example 1 except that 48 parts by mass of the polythiol composition of Example 1 were replaced by 48 parts by mass of the polythiol composition of Example 4 in Production Example 1. Physical properties were each determined based on each of the methods of evaluating physical properties of the plastic lens described above.

### [Production Example 5]

A plastic lens was produced by the same method as that described in Production Example 1 except that 48 parts by mass of the polythiol composition of Example 1 were replaced by 48 parts by mass of the polythiol composition of Example 5 in Production Example 1. Physical properties were each determined based on each of the methods of evaluating physical properties of the plastic lens described above.

### [Production Example 6]

A plastic lens was produced by the same method as that described in Production Example 1 except that 48 parts by mass of the polythiol composition of Example 1 were replaced by 48 parts by mass of the polythiol composition of Example 6 in Production Example 1. Physical properties were each determined based on each of the methods of evaluating physical properties of the plastic lens described above.

Physical properties of the plastic lenses of Production Examples 1 to 6 (that is, Examples 1 to 6) are shown in Table 1.

**[Table 1]**

| | | Example 1 | Example 2 | Example 3 | Example 4 | Example 5 | Example 6 |
|---|---|---|---|---|---|---|---|
| Polythiol composition | Proportion of peak area of compound represented by Formula (1) with respect to total peak area 100 of compounds contained in polythiol composition (area%) | 0.28 | 0.26 | 1.26 | 3.22 | 5.25 | 10.14 |
| | Proportion of peak area of compound represented by Formula (1) with respect to peak area 100 of polythiol compound (A) (area%) | 0.30 | 0.29 | 1.42 | 3.69 | 6.14 | 12.46 |
| | Proportion of peak area of polythiol compound (B) with respect to total peak area 100 of compounds contained in polythiol composition (area%) | 0.60 | 3.01 | 2.92 | 2.91 | 2.80 | 2.64 |
| | Proportion of peak area of polythiol compound (A) with respect to total peak area 100 of compounds contained in polythiol composition (area%) | 92.34 | 89.93 | 88.95 | 87.41 | 85.61 | 81.38 |
| Plastic lens | YI | 4.3 | 4.4 | 4.3 | 4.3 | 4.2 | 4.0 |
| | Degree of opacity | 21 | 26 | 24 | 23 | 22 | 20 |
| | Tg (°C) | 86.82 | 91.98 | 90.37 | 89.78 | 89.59 | 87.78 |

As shown in Table 1, the respective Examples, using a polythiol composition comprising: a polythiol compound (A) that was 4-mercaptomethyl-1,8-dimercapto-3,6-dithiaoctane; and a compound represented by the Formula (1), wherein, in a high-performance liquid chromatography measurement, a peak area of the compound represented by the Formula (1) was 11.0 or less with respect to a total peak area 100 of compounds contained in the polythiol composition, were excellent in the evaluations for yellow index and degree of opacity as well as heat resistance.

As a result, a resin having reduced yellow index and degree of opacity and being excellent in heat resistance was able to be produced.

Among them, Examples 1 to 5, using a polythiol composition, wherein the peak area of the compound represented by the Formula (1) was 10.0 or less with respect to a total peak area 100 of compounds contained in the polythiol composition, were more excellent in heat resistance.

### [Production Example 1X to Production Example 6X]

As each of Production Example 1X to Production Example 6X, a plastic lens was produced by performing the same procedure as that in each of Production Example 1 to Production Example 6 (that is, Example 1 to Example 4, Comparative Example 1 to Comparative Example 2) except for the following modifications, and substantially the same results as the results (Table 1) of each of Production Example 1 to Production Example 6 were obtained.

### - Modifications from Each of Production Example 1 to Production Example 6 -

In each of Production Example 1 to Production Example 6, m-xylylene diisocyanate (XDI) (52 parts by mass) was used when producing a plastic lens. Meanwhile, in each of Production Example 1X to Production Example 6X, the XDI (52 parts by mass) was changed to an XDI composition X1 (in an amount corresponding to 52 parts by mass of XDI being contained) as the XDI composition described above.

The XDI composition X1 was produced by adding a very small amount of the compound (N1), a very small amount of the compound (N2), and a very small amount of the compound (N3) to XDI that was a main component, and mixing them.

A gas chromatography measurement was performed for the XDI composition X1 under each of the GC conditions 1 and 2 described above, and as a result,
the peak area of the compound (N1) was 0.20 ppm or more (specifically, 600 ppm) with respect to the peak area 1 of XDI,
the peak area of the compound (N2) was 0.05 ppm or more (specifically, 18 ppm) with respect to the peak area 1 of XDI, and
the peak area of the compound (N3) was 0.10 ppm or more (specifically, 100 ppm) with respect to the peak area 1 of XDI.

The disclosure of Japanese Patent Application No. 2022-165813 filed on October 14, 2022 is incorporated herein by reference in its entirety.

All documents, patent applications, and technical standards described herein are incorporated herein by reference to the same extent as if each document, patent application, and technical standard were specifically and individually indicated to be incorporated by reference.

## Claims

1. A polythiol composition, comprising:
a polythiol compound (A) that is 4-mercaptomethyl-1,8-dimercapto-3,6-dithiaoctane; and
a compound represented by the following Formula (1): 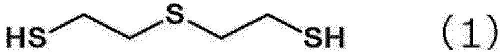
wherein, in a high-performance liquid chromatography measurement, a peak area of the compound represented by the Formula (1) is 11.00 or less with respect to a total peak area 100 of compounds contained in the polythiol composition.

2. The polythiol composition according to claim 1, wherein, in the high-performance liquid chromatography measurement, the peak area of the compound represented by the Formula (1) is 13.00 or less with respect to a peak area 100 of the polythiol compound (A).

3. The polythiol composition according to claim 2, wherein the peak area of the compound represented by the Formula (1) is 8.00 or less with respect to the peak area 100 of the polythiol compound (A).

4. The polythiol composition according to claim 1, further comprising a polythiol compound (B) comprising at least one selected from the group consisting of 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane.

5. The polythiol composition according to claim 4, wherein, in a high-performance liquid chromatography measurement, a peak area of the polythiol compound (B) is 3.10 or less with respect to a total peak area 100 of compounds contained in the polythiol composition.

6. A polymerizable composition, comprising:
the polythiol composition according to any one of claims 1 to 5; and
a polyiso(thio)cyanate compound.

7. The polymerizable composition according to claim 6, wherein the polyiso(thio)cyanate compound comprises at least one selected from the group consisting of pentamethylene diisocyanate, hexamethylene diisocyanate, xylylene diisocyanate, isophorone diisocyanate, bis(isocyanatomethyl)cyclohexane, bis(isocyanatocyclohexyl)methane, 2,5-bis(isocyanatomethyl)bicyclo-[2.2.1]-heptane, 2,6-bis(isocyanatomethyl)bicyclo-[2.2.1]-heptane, tolylene diisocyanate, 4,4'-diphenylmethane diisocyanate, and phenylene diisocyanate.

8. The polymerizable composition according to claim 6,
wherein the polymerizable composition comprises a polyiso(thio)cyanate composition comprising the polyiso(thio)cyanate compound, and
wherein the polyiso(thio)cyanate composition comprises: xylylene diisocyanate; and at least one selected from the group consisting of the following compound (N1), the following compound (N2), and the following compound (N3): 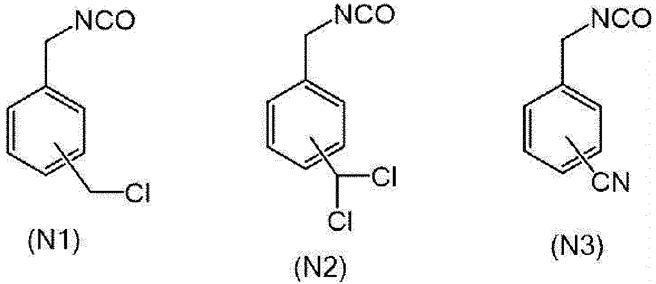 wherein:
in a case in which the polyiso(thio)cyanate composition comprises the compound (N1), a peak area of the compound (N1) is 0.20 ppm or more with respect to a peak area 100 of xylylene diisocyanate in a gas chromatography measurement,
in a case in which the polyiso(thio)cyanate composition comprises the compound (N2), a peak area of the compound (N2) is 0.05 ppm or more with respect to a peak area 100 of xylylene diisocyanate in a gas chromatography measurement, and
in a case in which the polyiso(thio)cyanate composition comprises the compound (N3), a peak area of the compound (N3) is 0.10 ppm or more with respect to a peak area 100 of xylylene diisocyanate in a gas chromatography measurement.

9. A resin, comprising a cured product of the polymerizable composition according to claim 6.

10. A molded body, comprising the resin according to claim 9.

11. An optical material, comprising the resin according to claim 9.

12. A lens, comprising the resin according to claim 9.
